(19) Europäisches Patentamt
European Patent Office
Office européen des brevets

(11) EP 4 478 050 A1

(12) # EUROPEAN PATENT APPLICATION

(43) Date of publication:
**18.12.2024 Bulletin 2024/51**

(21) Application number: **23178621.1**

(22) Date of filing: **12.06.2023**

(51) International Patent Classification (IPC):
***G01N 33/68** (2006.01)*

(52) Cooperative Patent Classification (CPC):
**G01N 33/6842;** G01N 2333/21; G01N 2333/245; G01N 2333/25

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Janssen Pharmaceuticals, Inc.**
**Titusville, NJ 08560 (US)**

(72) Inventors:
- **FLÜTSCH, Andreas**
  **3018 Bern (CH)**
- **BUFF, Rolf**
  **3018 Bern (CH)**

(74) Representative: **Beslier, Victor**
**Janssen Vaccines & Prevention B.V.**
**Archimedesweg 4-6**
**2333 CN Leiden (NL)**

(54) **METHOD FOR QUANTIFYING BOUND ACETATE IN GLYCOCONJUGATES**

(57) The present invention relates to a method for absolute quantification of covalently bound acetate of an acetylated glycoconjugate in a sample. Absolute quantification is performed using a colorimetric assay or a fluorometric assay based on a calibration curve.

EP 4 478 050 A1

## Description

The present invention relates to a method for absolute quantification of covalently bound acetate of an acetylated glycoconjugate in a sample. Absolute quantification is performed using a colorimetric assay or a fluorometric assay based on a calibration curve.

### Background of the invention

Glycoconjugates have found widespread application in medicine, in particular as glycoconjugate vaccines. Such glycoconjugate vaccines are produced by covalently linking a bacterial polysaccharide (PS), *e.g.*, O-antigen polysaccharide, to a carrier protein.

An example of a glycoconjugate vaccine are multivalent vaccines against extraintestinal pathogenic *Escherichia coli* (ExPEC).

In particular, efforts toward the development of a vaccine to prevent ExPEC infections have focused on multivalent compositions comprising bioconjugates of *E. coli* O-antigen polysaccharide covalently attached to a carrier protein [see e.g. Poolman and Wacker, J. Infect. Dis. (2016) v.213(1), pp. 6-13; WO 2015/124769; WO 2017/035181; WO2020/191082A1].

The O-antigen comprises the immunodominant component of the cell wall lipopolysaccharide (LPS) in Gram-negative bacteria, including *E. coli.* There are currently >180 serologically unique *E. coli* O-antigens identified, with the vast majority of ExPEC isolates classified within less than 20 O-antigen serotypes. Full-length *E. coli* O-antigen polysaccharides (O-PS) are typically comprised of about 10 to 25 repeating sugar units. In wild type *E. coli,* such O-PS are attached to the highly conserved LPS core structure. Each of the aforementioned components, i.e. O-PS component and LPS core structure, are synthesized separately by enzymes encoded predominantly in the rfb and rfa gene clusters, respectively. Following polymerization of the O-antigen, the O-PS backbone may be modified.

In particular, acetyl groups may be covalently linked to PS via oxygen and/or nitrogen. Such acetyl groups covalently bound to specific sugar moieties of polysaccharides, particularly *O*-acetyl groups, also referred to as acetoxy groups, can work as functional immunogenic epitopes *in vivo.* Accordingly, they are considered a critical quality attribute for glycoconjugate vaccines. However, absolute quantification of bound acetate of complex analytes, such as glycoconjugates, in particular polyvalent glycoconjugate vaccines such as vaccines against extra-intestinal pathogenic *E. coli* (ExPEC), *Shigella flexneri, Neisseria meningitides* or *Streptococcus pneumonia,* is a challenging task; and even more so if the determination method has to be suitable under pharmaceutical standards, *i.e.,* under "Good Laboratory Practice" (GLP) conditions, *e.g.,* for quality control, such as release control, for final drug products. Criteria that must be met by such determination method in order to be suitable under pharmaceutical standards are commonly referred to as System Suitability Criteria (SSC) and *inter alia* include those listed in table 1 below.

Table 1. Selected SSC

| **Criterion** | **Acceptance threshold** |
|---|---|
| Linearity of the calibration curve | Correlation coefficient of regression line $R^2 \geq 0.98$ |
| Linearity of the calibration curve | Deviation of individual measured calibration points from the nominal value $\leq \pm$ 10%, |
| Reproducibility | Relative standard deviation (% RSD) of duplicate determination of samples $\leq$ 15% |

The current gold standard for quantifying covalently bound acetate of glycoconjugates relies on high-performance anion-exchange chromatography with conductivity detection (HPAEC-CD) [e.g. Kao et al, Vaccine, 2004 Jan 2;22(3-4):335-44]. However, HPAEC-CD generally suffers from relatively low throughput and relatively long analysis time. In particular, throughput of HPAEC-CD based methods for acetate quantification is typically limited to the analysis of about six samples within 18 h. Furthermore, sensitivity of currently available HPAEC-CD based methods - although representing the currently most sensitive analysis method - is not sufficient for certain sample types, such as certain multivalent drug products containing a multitude of glycoconjugates. In certain multivalent drug products, *e.g.,* polyvalent ExPEC glycoconjugate vaccines, the concentration of acetylated glycoconjugates - which is typically only a fraction of all different glycoconjugates present in such sample - and thereby the concentration of bound acetyl groups, is sometimes below the sensitivity of common HPAEC-CD methods.

In particular, sensitivity of current HPAEC-CD methods is in the range of about 7 ng of acetate ion [Kao et al, Vaccine, 2004 Jan 2;22(3-4):335-44], which corresponds to about 12 $\mu$M acetate in 10 $\mu$l final sample volume. However, given that several sample preparation steps are required to arrive at the final sample volume, a higher concentration of

bound *O*-acetate is typically required in the starting material, such as a drug product containing a mixture of glycoconjugates. In addition, comparing sensitivities of different literature methods is challenging since sometimes it is not clear from the literature whether a stated "sensitivity" value refers to the limit of detection (LOD) or the limit of quantification (LOQ).

In addition, Hestrin et al. [J Biol Chem 1949; 180:249-61] have described a method for quantifying acetylcholine based on non-enzymatic colorimetric acetate quantification. However, sensitivity of the non-enzymatic colorimetric method has been found to be 10 - 20 fold lower than that of HPAEC-CD [Kao et al, Vaccine, 2004 Jan 2;22(3-4):335-44].

Furthermore, $^{13}C$ nuclear magnetic resonance (NMR) has been leveraged to determine bound acetate of certain glycoconjugates [e.g. Bundle et al, J Biol Chem 1974;249(7):2275-81 or Bhattacharjee et al, Can J Biochem 1976;54(1):1-8]. However, although $^{13}C$ NMR spectroscopy may be used to determine bound acetate of glycoconjugates, it suffers from several disadvantages, in particular a relatively low throughput, the requirement for large amount of analyte and relatively high costs.

In consequence, there is a need for an improved method for absolute quantification of covalently bound acetate of acetylated glycoconjugates. In particular, there is a need to provide an analysis method having a higher throughput than the current gold standard method, *i.e.,* HPAEC-CD. Further, there is a need to provide an analytical method having a better sensitivity (sensitivity being defined as the LOQ) than currently available methods.

Moreover, there is a need to provide a method that provides results within a short period of time, in particular such that analysis results are available within one working day, *i.e.,* within less than 8 h. In addition, there is a need to provide a method which is robust and can be reproducibly operated under GLP conditions satisfying the SSC criteria listed in table 1 in an economically feasible manner.

## Description of the invention

The above objectives are achieved by a method as defined in claim 1. Further aspects of the invention are disclosed in the specification and the dependent claims. An illustrative (non-limiting) workflow is shown in Fig. 1.

The present invention will be described in more detail below. It is understood that the various embodiments, preferences and ranges as provided / disclosed in this specification may be combined at will. Further, depending on the specific embodiment, selected definitions, embodiments or ranges may not apply.

Throughout this specification a number of abbreviations are used, including:

| | |
|---|---|
| ATP | Adenosine triphosphate |
| DOA | Degree of acetylation |
| EPA | ExoProtein A (also referred to as exotoxin A) of *Pseudomonas aeruginosa* |
| *E. coli* | *Escherichia coli* |
| ExPEC | Extra-intestinal pathogenic *E. coli* |
| HPAEC-CD | High-performance anion-exchange chromatography with conductivity detection |
| HPLC | High-Performance Liquid Chromatography |
| IED | Invasive ExPEC disease |
| LOD | limit of detection |
| LOQ | limit of quantification |
| MWCO | molecular weight cut-off |
| O-EPA | glycoconjugate of EPA carrier protein covalently coupled to bacterial O-antigen polysaccharide |
| *P. aeruginosa* | *Pseudomonas aeruginosa* |
| PS | polysaccharide |
| RSD | relative standard deviation |
| RT | room temperature (also referred to as ambient temperature; about 20°C) |
| RU | repeating unit |
| S/N | signal-to-noise ratio |
| SSC | System Suitability Criteria |
| TFA | Trifluoroacetic acid |

Unless otherwise stated, the following definitions shall apply in this specification:
As used herein, the term "a", "an", "the" and similar terms used in the context of the present invention (especially in the context of the claims) are to be construed to cover both the singular and plural unless otherwise indicated herein or clearly contradicted by the context.

As used herein, the terms "including", "containing" and "comprising" are used herein in their open, non-limiting sense. It is understood that the various embodiments, preferences and ranges may be combined at will.

The term **"glycoconjugate"** is known in the field and particularly describes chemical entities covalently bound to one or more polysaccharide(s). Such glycoconjugate may be obtained by biological conjugation in a living cell ("bioconjugate" or "biological conjugate") or may be obtained by chemical conjugation of a polysaccharide ("chemical" or "synthetic" glycoconjugate). Particularly suitable chemical entities are proteins, the corresponding glycoconjugates being glycoproteins. Specifically, the term glycoconjugates relates to a conjugation product wherein a polysaccharide (i.e. a glycan) is covalently coupled to a carrier protein. Examples of such glycoconjugates include "glycoconjugate vaccines". The skilled person understands that in glycoconjugate vaccines, the emphasis is on the glycan part, to which an immune response is desired because the glycans are the relevant antigens, and the protein part merely serves as a carrier to lead to a desired T-cell memory immune response.

If the carrier protein of glycoconjugate comprises more than one glycosylation sites, mixtures of glycoconjugates, such as bioconjugates, are obtained, e.g. mixtures of mono-, di-, tri-, and tetra-glycosylated bioconjugates. An example of a carrier protein comprising 4 glycosylation site is an EPA carrier protein having the amino acid sequence of SEQ ID NO: 1 of WO 2022/214620. "Bioconjugate compositions" comprise such mixtures, and these may include bioconjugates of one polysaccharide (e.g. as in drug substances of a glycoconjugate of a carrier protein coupled to O-antigen polysaccharide of a specific bacterial serotype; sometimes referred to as "monovalent composition") or alternatively of more than one polysaccharide (e.g. as in a drug product wherein O-antigen polysaccharides of multiple bacterial serotypes each individually coupled to carrier proteins are included in the same composition; sometimes referred to as "multivalent composition").

The term "**bioconjugate**" is known in the field and specifically relates to a glycoconjugate prepared in a host cell, wherein the host cell machinery produces the glycan and the carrier protein and links the glycan to the carrier protein, e.g., via N-links of asparagine or arginine. A particularly preferred host cell for producing bioconjugates is *E. coli,* preferably comprising nucleic acid encoding: (i) the carrier protein, (ii) an oligosaccharyltransferase such as *C. jejuni* PglB that is capable of covalently linking O-antigen polysaccharides to an asparagine (Asn) residue in a glycosylation consensus sequence (e.g. Asn-X-Ser(Thr), wherein X can be any amino acid except Pro) in a carrier protein via N-linked glycosylation. In certain embodiments, the host cell, preferably *E. coli,* further comprises nucleic acid encoding (iii) an *rfb* gene cluster encoding the enzymes responsible for generating the O-antigen polysaccharide of a desired serotype, e.g. a desired *E. coli* serotype. By creating host cells with a different *rfb* locus, different bioconjugates can be prepared, e.g. comprising O-antigen polysaccharides from different *E. coli* or *Shigella* serotypes. Culturing such host cells will produce the bioconjugates comprising the carrier protein to which the O-antigen polysaccharide encoded by the *rfb* locus is covalently attached, within the periplasm of the host cell. A more detailed description for production of bioconjugates in such host cells can for instance be found in WO 2009/104074, WO 2015/124769, WO 2017/035181, or WO 2020/191082.

The term "**carrier protein**" is known in the field and relates to a protein to which one or more polysaccharides are covalently bound. A particularly suitable carrier protein in the context of the invention is detoxified Exoprotein A of *Pseudomonas aeruginosa* (EPA). As is common in the field, the terms Exotoxin A and ExoProtein A of *P. aeruginosa,* or EPA, may be used interchangeably. In particular embodiments, a carrier protein is a detoxified Exoprotein A of *P. aeruginosa.* For EPA, various detoxified protein variants have been described in literature and could be used as carrier proteins. For example, detoxification can be achieved by mutating and deleting the catalytically essential residues L552V and ΔE553.

In certain embodiments, the EPA carrier protein comprises 1 to 20, preferably 1 to 10, more preferably 2 to 4, glycosylation sites.

In certain embodiments, the EPA comprises four glycosylation sites having the amino acid sequence Asp(Glu)-X-Asn-Z-Ser(Thr), wherein X and Z are independently selected from any amino acid except Pro, such as an EPA carrier protein comprising the amino acid sequence of SEQ ID NO: 1 of WO 2022/214620. See for example WO 2015/124769, WO 2017/035181, or WO 2020/191082 for a description of examples of bioconjugation of *E.coli* O-antigen polysaccharides to EPA carrier protein, or for example WO 2009/104074 for a description of examples of bioconjugation of *Shigella* O-antigen polysaccharides to EPA carrier protein. In one embodiment, the carrier protein comprises an amino acid sequence with at least 80%, 85%, 90%, 95%, 96%, 97%, 98% or 99% sequence identity to SEQ ID NO: 1 of WO 2022/214620. In certain embodiments, the carrier protein of a bioconjugate according to the invention comprises SEQ ID NO: 1 of WO 2022/214620.

The terms "**percent (%) sequence identity**" or "**% identity**" are known in the field and relate to the number of matches ("hits") of identical amino acids of two or more aligned amino acid sequences as compared to the number of amino acid residues making up the overall length of the amino acid sequences. The percent identity of two amino acid sequences is thus the number of exact matches between two aligned sequences divided by the length of the shorter sequences and multiplied by 100. The sequences which are compared to determine sequence identity may thus differ by substitution(s), addition(s) or deletion(s) of amino acids. Suitable programs for aligning amino acid sequences are known to the skilled person.

The term "**polysaccharide**" is known in the field and describes polymeric carbohydrates composed of monosaccharide units bound together by glycosidic linkages, either linear or branched. Such polysaccharides are characterized by their repeating units (RU), each repeating unit described with their respective monosaccharide composition. Said repeating units include one or more monosaccharides which can also be modified (e.g. amidated, sulphonated,

acetylated, phosphorylated, etc). Typically found monosaccharides in said repeating units are cyclic or linear monosaccharides containing three to seven carbon atoms. In the specific case of glycoconjugate vaccines, the conjugated polysaccharide originates from a pathogenic species (e.g. ExPEC or *Shigella*) with said repeating unit defined by the genetics of the specific pathogen. The repeating unit can thus be a specific marker / identifier of the pathogen.

In the context of the present invention, suitable polysaccharides typically comprise 1 to 100, such as 1-50, 1-40, 1-30, 1-20, 1-10, 3-50, 3-40, 5 - 20, 10 - 20, repeating units n. Such repeating units contain (i.e. comprise or consist of) (i) non-modified monosaccharides and/or (ii) modified monosaccharides. The term "modified mono-saccharides" in non-limiting embodiments includes *N*-acetylation, *O*-acetylation, amidation and/or amination of mono-saccharides, particularly *O*-acetylation. Such modified mono-saccharides may comprise zero, one, or more modifications, for example zero, one, two or three of the above modifications, at the same mono-saccharide.

In particular embodiments, modified monosaccharides are *O*-acetylated and/or *N*-acetylated monosaccharides, particularly *O*-acetylated, such as monosaccharides comprising one *O*-acetylation.

In embodiments of the invention, suitable repeating units comprise mono-saccharides selected from the group consisting of Mannose, Rhamnose, Glucose, Fucose, Galactose, modified Mannose, modified Rhamnose, modified Glucose, modified Fucose, and modified Galactose.

In certain embodiments, the *O*-polysaccharide is specific to a Gram-negative bacterium selected from the list of *Escherichia* and *Shigella,* preferably *E. coli.*

Non-limiting and exemplary structures of *E. coli O*-antigen polysaccharides are shown below in table 2. A single RU for each *E. coli O*-antigen polysaccharide is shown. In this table, each n is independently an integer of 1 to 100, such as 1-50, 1-40, 1-30, 1-20, and 1-10, 3-50, 3-40, e.g. at least 5, such as 5-40, e.g. 7-30, e.g. 7 to 25, e.g. 5 to 20, e.g. 10 - 20, but in some instances can be 1-2.

The terms **"repeating unit"** or "RU" are known in the field and as used with respect to an O-antigen refer to the biological repeat unit of an O-antigen as it is synthesized *in vivo* by cellular machinery (e.g., glycosyltransferases).

The terms **"polysaccharide component"** or **"PS-component"** consequently denote one or more glycan chain(s) of a glycoconjugate. Glycans can be monomers or polymers of sugar residues, but typically contain at least three sugars, and can be linear or branched. A glycan may include natural sugar residues (e.g., glucose, *N*-acetylglucosamine, *N*-acetyl neuraminic acid, galactose, mannose, fucose, arabinose, ribose, xylose, etc.) and/or modified sugars (e.g., 2'-fluororibose, 2'-deoxyribose, phosphomannose, 6'-sulfo *N*-acetylglucosamine, etc). The term "glycan" includes homo- and heteropolymers of sugar residues. The term "glycan" also encompasses a glycan component of a glycoconjugate (e.g., of a glycoprotein, glycopeptide).

The term **"*O*-acetylated polysaccharide",** as used herein, refers to polysaccharides where one or more monosaccharides of the repeating unit are modified by acetylation via oxygen, the resulting functionality being referred to as *O*-acetyl, *O*-acetate or acetoxy groups. Said monosaccharides have one or more of their present hydroxyl groups acetylated. In certain embodiments, the *O*-acetylated PS is acetylated at Rhamnose. For pathogen-derived repeating units used in glycoconjugate vaccines, the *O*-acetylation of certain monosaccharides can influence induction of an immune response for said pathogen. Examples of pathogen-derived polysaccharide components including *E. coli* 016 antigen PS and *E. coli* O25B antigen PS, are shown in table 2.

The terms "**glycan**" / "**glycan chain**" are synonyms of "polysaccharide" as defined above. Correspondingly, in the context of this invention, "glycan" and the prefix "glyco", also refers to the carbohydrate portion of a glycoconjugate, such as a glycoprotein. Accordingly, "glycosylated" refers to one or more polysaccharides covalently attached to a protein, thus the protein being "monoglycosylated" or "multiglycosylated", respectively.

The term "**serotype**" as used herein, refers to glycoconjugates having different polysaccharide chains which are derived from different bacterial serotypes. Examples of glycans from a number of *E. coli* serotypes are identified below in table 2.

The term "**drug substance**" as used herein refers to the bulk product of an individual glycoconjugate, such as a bioconjugate (e.g. *E. coli* O-antigen polysaccharide covalently coupled to EPA carrier protein, e.g. *E. coli* O25B O-antigen or *E. coli* 016 O-antigen covalently coupled to EPA), that is at higher concentration than the product as will be finally administered to a subject in need thereof. The drug substance can be produced after purification of the glycoconjugate, particularly the bioconjugate. The drug substance can, for example, be stored in a more concentrated form in a suitable formulation buffer (see e.g. WO 2018/077853, WO2020/191082), for instance in frozen condition, e.g. at minus 70° C.

The term "**drug product**" as used herein refers to the formulation of the glycoconjugates, e.g. *E. coli* O-antigen polysaccharides individually coupled to EPA carrier protein, in final form for administration to a subject in need thereof. In certain embodiments, the sample is a drug product containing a multivalent glycoconjugate vaccine composition, e.g. a four-valent ExPEC glycoconjugate vaccine composition comprising the *E. coli* O-antigen polysaccharides O25B, O1A, O2 and O6A, each individually coupled to an EPA carrier protein. Further non-limiting examples of multivalent glycoconjugate vaccine compositions (samples) are, *e.g.,* a nine-valent glycoconjugate vaccine compositions comprising the *E. coli* O-antigen polysaccharides O1A, O2, O4, O6A, 015, 016, O18A, O25B and O75, and, *e.g.,* a ten-valent glycoconjugate vaccine composition comprising the *E. coli* O-antigen polysaccharides O1A, O2, O4, O6A, O8, 015, 016, O18A, O25B and

O75, each *E. coli* O-antigen polysaccharide being individually coupled to an EPA carrier protein (see e.g. WO2020/191082 and WO2022/058945 for examples of ten- and nine-valent glycoconjugate vaccine compositions, respectively). In certain embodiments, multivalent glycoconjugate vaccine compositions may include additional *E. coli* O-antigen polysaccharides, individually coupled to EPA carrier protein. Based on the present disclosure, the skilled person understands that further glycoconjugates may be added to the sample without disturbing the inventive method described herein. Drug product can typically be prepared by mixing the drug substances of the respective glycoconjugates, and dilution by a suitable formulation buffer if needed (see e.g. WO2018/077853, WO2020/191082, WO2022/058945), such that the target dose of vaccine is produced.

Table 2. Examples of structures of *E. coli* O-antigen polysaccharides

| *E. coli* O-antigen Polysaccharide<br>Structure of Repeating Unit |
|---|
| O1A antigen polysaccharide (O1A) |
| [→3)-α-L-Rha*p*-(1→3)-α-L-Rha*p*-(1→3)-β-L-Rha*p*-(1→4)-β-D-Glc*p*NAc-(1→]$_n$<br>2<br>↑<br>1<br>β-D-Man*p*NAc |
| 02 antigen polysaccharide (02) |
| [→3)-α-L-Rha*p*-(1→2)-α-L-Rha*p*-(1→3)-β-L-Rha*p*-(1→4)-β-D-Glc*p*NAc-(1→]$_n$<br>2<br>↑<br>1<br>α-D-Fuc*p*3NAc |
| 04 antigen polysaccharide (04) |
| α-D-Glc*p*<br>1<br>↓<br>3<br>[→2)-α-L-Rha*p*-(1→6)-α-D-Glc*p*-(1→3)-α-L-Fuc*p*NAc-(1→3)-β-D-Glc*p*NAc-(1→]$_n$ |
| O6A antigen polysaccharide (06) |
| [→4)-α-D-Gal*p*NAc-(1→3)-β-D-Man*p*-(1→4)-β-D-Man*p*-(1→3)-α-D-Glc*p*NAc-(1→]$_n$<br>2<br>↑<br>1<br>β-D-Glc*p* |
| 08 antigen polysaccharide (08)<br>**α-D-Man*p*3Me-(1→[3)-β-D-Man*p*-(1→2)-a-D-Man*p*-(1→2)-α-D-Manp-(1→]$_n$** |
| 015 antigen polysaccharide (015)<br>**[→2)-β-D-Gal*p*-(1→3)-α-L-Fuc*p*NAc-(1→3)-β-D-Glc*p*NAc-(1→]$_n$** |
| 016 antigen polysaccharide (016) |
| [→2)-β-D-Gal*f*-(1→6)-α-D-Glc*p*-(1→3)-α-L-Rha*p*-(1→3)-α-D-Glc*p*NAc-(1→]$_n$<br>2<br>↑<br>Ac |
| O18A antigen polysaccharide (O18A) |

(continued)

| *E. coli* O-antigen Polysaccharide<br>Structure of Repeating Unit |
|---|
| [→2)-α-L-Rha*p*-(1→6)-α-D-Glc*p*-(1→4)-α-D-Gal*p*-(1→3)-α-D-Glc*p*NAc-(1→]$_n$<br>3<br>↑<br>1<br>β-D-Glc*p*NAc |
| O25B antigen polysaccharide (O25B)<br>β-D-Glc*p*<br>1<br>↓<br>6<br>[→4)-α-D-Glc*p*-(1→3)-α-L-Rha*p*-(1→3)-β-D-Glc*p*NAc-(1→]$_n$<br>3 2<br>↑ ↑<br>1 Ac<br>α-L-Rha*p* |
| 075 antigen polysaccharide (075)<br>β-D-Man*p*<br>1<br>↓<br>4<br>[→3)-α-D-Gal*p*-(1→4)-α-L-Rha*p*-(1→3)-β-D-Glc*p*NAc-(1→]$_n$ |

All monosaccharides described herein (e.g., table 2) have the common meaning known in the art. In general, monosaccharides can have the D or L configuration. If D or L is not specified, the monosaccharide is understood to have the D configuration. Monosaccharides are typically referred to by abbreviations commonly known and used in the art. For example, Glc refers to glucose; D-Glc refers to D-glucose; and L-Glc refers to L-glucose. Other common abbreviations for monosaccharides used in table 2 include: Rha, rhamnose; GlcNAc, N-acetylglucosamine; GalNAc, Nacetylgalactosamine; Fuc, fucose; Man, mannose; Man3Me, 3-O-methyl-mannose; Gal, galactose; FucNAc, N-acetylfucosamine; Rib, ribose; Ac represents acetyl, whereby Ac in combination with an arrow indicates an *O*-acetylation site (acetoxy, *O*-acetate; see 016 and O25B repeating units) and NAc represents *N*-acetyl. The suffix "f' refers to furanose and the suffix "p" refers to pyranose. For example, GlcpNAc indicates *N*-acetylglucosamine in pyranose form).

The invention relates to a method for absolute quantification of covalently bound acetate, particularly *O*-acetate, of an acetylated glycoconjugate, *e.g.*, an acetylated bioconjugate, in a sample (also referred to as "test sample"). The acetylated glycoconjugate, *e.g.,* the acetylated bioconjugate, comprises one carrier protein and one or more polysaccharides covalently bound to said carrier protein, wherein one or more acetate groups are covalently bound to oxygen of the one or more polysaccharides. The inventive method comprises the following steps:

a) Providing a sample containing the acetylated glycoconjugate; then
b) precipitating the acetylated glycoconjugate, to thereby obtain a pellet containing the acetylated glycoconjugate; then
c) hydrolysing *O*-acetate linkages of the acetylated glycoconjugate contained in the pellet, to thereby release covalently bound *O*-acetate; then
d) absolute quantification of the released acetate using a colorimetric assay or a fluorometric assay based on a calibration curve.

As the skilled person understands based on the present disclosure, the acetylated glycoconjugate may, in addition to *O*-acetate linkages, further contain *N*-acetate linkages (i.e. one or more acetate groups being covalently bound to nitrogen of the one or more polysaccharides), which may also be quantified by the inventive method.

This shall be explained in more detail below:

Carrier protein:

In certain embodiments, the carrier protein is selected from the group consisting of detoxified Exoprotein A of *Pseudomonas aeruginosa* (EPA), *Escherichia coli* flagellin (FliC), CRM197, maltose binding protein (MBP), Diphtheria toxoid, Tetanus toxoid, detoxified hemolysin A of *Staphylococcus aureus,* clumping factor A, clumping factor B, *Escherichia coli* heat labile enterotoxin, detoxified variants of *Escherichia coli* heat labile enterotoxin, Cholera toxin B subunit (CTB), cholera toxin, detoxified variants of cholera toxin, *Escherichia coli* Sat protein, the passenger domain of *Escherichia coli* Sat protein, *Streptococcus pneumoniae* Pneumolysin, Keyhole limpet hemocyanin (KLH), *Pseudomonas aeruginosa* PcrV, outer membrane protein of *Neisseria meningitides* (OMPC), and protein D from non-typeable Haemophilus influenza.
In certain embodiments, the carrier protein is a detoxified Exoprotein A of *Pseudomonas aeruginosa* (EPA).
In certain embodiments, the carrier protein comprises an amino acid sequence with at least 80%, 85%, 90%, 95%, 96%, 97%, 98% or 99% sequence identity to SEQ ID NO: 1 of WO 2022/214620. In certain embodiments, the carrier protein is recombinant EPA that comprises the amino acid sequence of SEQ ID NO: 1 of WO 2022/214620.

Polysaccharide:

In certain embodiments, the one or more polysaccharides are O-antigen polysaccharides specific to a Gram-negative bacterium, preferably selected from the group consisting of *Escherichia* and *Shigella,* more preferably *E. coli.*
In certain embodiments, the one or more polysaccharides each comprise 1-100, preferably 5 - 20, repeating units.
In certain embodiments, the polysaccharide is an *E. coli* O-antigen polysaccharide selected from *E. coli* serotypes 016 and O25B. *E. coli* O-antigen polysaccharide of serotypes 016 and O25B are *O*-acetylated (cf. table 2 above; the acetylation site being indicated by an arrow and abbreviated as "Ac"). Further, a multivalent glycoconjugate vaccine against ExPEC containing O-EPA glycoconjugates comprising *E. coli* 016 antigen polysaccharide and *E. coli* O25B O-antigen polysaccharide is currently under development (see e.g. WO2020/191082 and WO2022/058945). Accordingly, a method for absolute quantification of covalently bound acetate of glycoconjugates, particularly glycoconjugates of *E. coli* 016 antigen polysaccharide and/or *E. coli* O25B O-antigen polysaccharide, is of particular relevance for developing glycoconjugate vaccines against ExPEC.

Step a, sample:

The term sample is known in the field and as used herein particularly relates to an aqueous sample. An aqueous sample generally includes any material comprising water which, optionally after dilution, may be supplied to an analytical system. In the context of the present invention, an aqueous sample comprises at least 10% (w/w) water, e.g. 10% - 99% (w/w) water, e.g. 50-99% (w/w), such as 60% - 99% (w/w), 80% - 99% (w/w), 70 - 95% (w/w) water. Such samples particularly include production batches of glycoconjugates, e.g. bioconjugates, (including in-process batches and stored production batches) and compositions comprising multiple glycoconjugates, such as pharmaceutical compositions (drug product) comprising a multivalent glycoconjugate vaccine.
The sample contains at least one acetylated glycoconjugate, such as a bioconjugate. Examples of such acetylated glycoconjugates include, but are not limited to, O-EPA glycoconjugates comprising a carrier protein as described above and one or more polysaccharides selected from *E. coli* 016 antigen polysaccharide (O16-EPA conjugate) and *E. coli* O25B O-antigen polysaccharide (O25B-EPA conjugate). In particular, the sample contains the acetylated glycoconjugates in dispersed form. In certain embodiments, the sample is a clear (aqueous) solution and, accordingly, contains the acetylated glycoconjugate in dissolved form. In certain embodiments, the sample is a suspension, i.e. it contains the acetylated glycoconjugate or another component of the sample in suspended form. In certain embodiments, the sample is a colloidal solution, i.e. it contains the acetylated glycoconjugate or another component of the sample as microscopically dispersed particles that are suspended throughout the liquid phase of the sample. In certain embodiments, the sample is an emulsion, *e.g.,* comprising an aqueous phase and a hydrophobic phase such as silicon oil.
In certain embodiments, the concentration of the acetylated glycoconjugate in the sample is known. The inventive method thus enables determining the degree of acetylation (DOA) of an acetylated glycoconjugate based on the known concentration of the acetylated glycoconjugate and absolute quantification of bound acetate, particularly bound *O*-acetate. As is common in the field, the concentration of a glycoconjugate, e.g. a bioconjugate, in a sample may be determined based on absolute quantification of the polysaccharide component of said glycoconjugate. For example, the concentration of the acetylated glycoconjugate may be determined by LC-MS as described in WO2021219530. Alternatively, the concentration of the acetylated glycoconjugate may be determined by total acidic hydrolysis of the

polysaccharides into monosaccharides, followed by high performance anion exchange chromatography with pulsed amperometric detection (HPAEC-PAD) [see, *e.g.,* J. S. Rohrer, Carbohydrate Analysis by Modern Liquid Phase Separation Techniques (Second Edition) 2021, Pages 157-207].

An advantage of the inventive method is an increased "sensitivity", which is defined herein as the limit of quantification (LOQ). As is common in the field, the LOQ is defined as the lowest analyte concentration, *i.e.,* acetate covalently bound to oxygen of a PS component, that can be reliably quantified. However, the LOQ is not to be confused with the limit of detection (LOD) or detection limit. The LOD is the lowest analyte concentration, which can be distinguished from the absence of that analyte (blank value). As a rule of thumb, the LOQ can be approximated by multiplying the LOD by 3.3.

In the context of the present invention, unless otherwise indicated (cf. comparative example 2, LOQ for HPAEC-CD based acetate quantification), the LOQ is defined as the lowest concentration of covalently bound *O*-acetate in a sample containing an acetylated glycoconjugate for which the recovery rate of released acetate is at least 85%. Determining a recovery rate is within the ordinary skill. For example, the recovery rate may be determined based on absolute quantification of bound *O*-acetate (released acetate upon hydrolysis of *O*-acetate linkages) in a test sample containing the acetylated glycoconjugate of interest via an orthogonal test method, e.g., HPAEC-CD as described in example 2 or Kao et al. [Vaccine, 2004 Jan 2;22(3-4):335-44], wherein the sample contains a bound *O*-acetate concentration that is within the linear range of the orthogonal test method. This test sample may then be further diluted to obtain diluted test samples having a known concentration of bound *O*-acetate, wherein the concentration is lower than the LOQ of the orthogonal test method. The recovery rate may then be determined by measuring the concentration of bound *O*-acetate using the inventive method described herein and comparing it with the concentration determined via the orthogonal test method, taking into account the dilution factor.

Alternatively, the recovery rate may be calculated by determining the bound *O*-acetate concentration according to the inventive method described herein and comparing it to the bound *O*-acetate concentration determined, also using the inventive method described herein, for an otherwise identical sample comprising a 5 times higher concentration of the acetylated glycoconjugate, taken into account the dilution factor. For example, the LOQ may be determined as follows:

- Determining the concentration of bound *O*-acetate (released acetate) using a first test sample containing a known concentration of the acetylated glycoconjugate (concentration based on the PS component) according to the method described herein;
- Diluting the first test sample by a factor of 5 using the same matrix (i.e., using the same composition as the first test sample but without the acetylated glycoconjugate) to thereby obtain a second test sample;
- Determining the concentration of bound *O*-acetate (released acetate) using the second test sample according to the method described herein;
- Comparing the concentration of bound *O*-acetate (released acetate) between the first test sample and the second (diluted) test sample;
- If the concentration of released acetate determined for the second test sample corresponds to at least 85% of released acetate determined for the first test sample (taking into account the dilution factor), the concentration of released acetate determined for the second (diluted) test sample is considered quantifiable (LOQ being the lowest concentration of bound acetate, particularly bound *O*-acetate that is quantifiable).

In certain embodiments, the LOQ of the inventive method was found to be about 8 μM bound *O*-acetate (concentration of bound *O*-acetate in the test sample; particularly measured with a precision of 4% relative standard deviation (RSD) and an accuracy of 102% recovery; cf. example 1). Nevertheless, in certain embodiments, the LOQ may also be lower than about 8 μM bound *O*-acetate.

In certain embodiments, the sample contains at least 8 μM bound acetate, particularly bound *O*-acetate, such as a bound *O*-acetate concentration of between 8 μM to 600 μM, *e.g.,* between 8 μM and 53 μM, such as between 8 μM and 19 μM. The skilled person understands that the concentration of acetylated glycoconjugate (as measured based on the quantification of the polysaccharide component of said glycoconjugate) that corresponds to a bound acetate, particularly bound *O*-acetate, concentration of between 8 μM to 600 μM varies dependent on the DOA (and thus dependent on the number of RUs as each RU may be *O*-acetylated; calculation of DOA will be explained in more detail in the section concerning optional "step e"). For example, a concentration of 8 μM bound *O*-acetate corresponds to a concentration of about 6 μg/ml of a glycoconjugate (measured as μg/ml of the PS component) comprising an EPA carrier protein glycosylated with one *E. coli* 016 antigen polysaccharide comprising, wherein each RU contains one *O*-acetyl group (i.e., DOA = 100%; molecular weight of *E. coli* 016 PS RU = 715.7 g/mol) or to a concentration of about 7 μg/ml of a glycoconjugate (measured as μg/ml of the PS component) comprising an EPA carrier protein glycosylated with one *E. coli* O25 antigen polysaccharide, wherein each RU contains one O-acetyl group (*i.e.,* DOA = 100%, molecular weight of *E. coli* O25B PS RU = 861.8 g/mol; RU structures shown in table 2).

For comparison, LOQ for absolute quantification of acetate using an HPAEC-CD based method under the strict SSC according to table 1 was found to be about 2.5 ng/μl bound *O*-acetate in the starting material, *i.e.,* the concentration of

bound *O*-acetate in the test sample prior to sample preparation (cf. example 2). This corresponds to a concentration of bound *O*-acetate in the test sample of about 42 μM.

However, a concentration of bound *O*-acetate of less than 42 μM may be present in certain test samples containing only low concentration of acetylated glycoconjugate(s). Further, a lower limit of quantification may be required in order to reliably quantify bound acetate in certain test samples, *e.g.,* test samples that are stored under stress conditions, *e.g.,* at elevated temperature, such as temperature > 8°C.

Additional components of the sample

The sample may, and typically does, contain additional components (i.e. in addition to the at least one acetylated glycoconjugate). For example, the sample may contain an aqueous matrix that optionally includes one or more of buffers, in-organic salts, free acetate, sugar alcohols, and non-ionic surfactants.

In particular, the sample may contain free acetate that has been cleaved off from the at least one acetylated glycoconjugate, *e.g.,* during storage. In addition, free acetate may be a component of a buffer, such as a sodium acetate buffer.

However, the presence of free acetate in the sample does not impede quantification of bound acetate, particularly bound *O*-acetate, according to the inventive method described herein. In particular, free acetate, if present, is removed during step b), precipitating the acetylated glycoconjugate, to thereby obtain a pellet containing the acetylated glycoconjugate.

In certain embodiments, particularly in the context of glycoconjugate vaccines, the sample typically comprises one or more buffers, in particular pharmaceutically acceptable buffers. An example of a pharmaceutically acceptable buffer that is suitable in the present invention is a phosphate buffer, e.g. a 10 mM phosphate buffer, particularly comprising 6.19 mM $KH_2PO_4$ and 3.81 mM $Na_2HPO_4$ at pH 7.0 (see e.g. WO 2018/077853).

In certain embodiments, the aqueous matrix comprises inorganic salts (*e.g.* NaCl), sugar alcohols (*e.g.* D-Sorbitol), and/or non-ionic surfactants (*e.g.* Polysorbate 80).

An exemplary aqueous matrix in the context of glycoconjugate vaccine comprises 6.19 mM $KH_2PO_4$, 3.81 mM $Na_2HPO_4$, 5% (w/w) sorbitol, 10 mM methionine, and 0.02% (w/w) polysorbate-80 at pH 7.0 (see e.g. WO 2018/077853).

In certain embodiments, the sample comprises carrier protein free of PS (also referred to as free carrier protein). Such free carrier protein may, *e.g.*, result from the cleavage of PS component from glycoconjugate(s) in the sample during storage or may be present as an impurity from the production process.

In certain embodiments, the sample comprises polysaccharides not bound to carrier protein ("free PS"). In analogy to free carrier protein, such free PS may, *e.g.,* result from the cleavage of PS component from glycoconjugate(s) in the sample during storage or may be present as an impurity from the production process.

In certain embodiments, the sample comprises non-related proteins, *e.g.,* host cell proteins that are present as an impurity from the production process.

In certain embodiments, the sample may comprise impurities, e.g. process-related impurities including free carrier protein, free PS and/or non-related proteins, such as host cell proteins. In certain embodiments, the aqueous sample may include up to 10% (w/w), up to 50% (w/w) or even up to 90% (w/w) impurities.

It is considered particularly beneficial that the inventive method is tolerant to a wide variety of additional components in the sample.

In certain embodiments, the sample comprises one single glycoconjugate, wherein this single glycoconjugate is an acetylated glycoconjugate.

In other embodiments, the sample comprises a multitude of different glycoconjugates, such as 2 - 20, *e.g.,* 4 - 10 different glycoconjugates, said different glycoconjugates differing in the polysaccharide components and/or in the carrier proteins. In these embodiments, at least one of the glycoconjugates is an acetylated glycoconjugate.

In certain embodiments, the sample comprises one single acetylated glycoconjugate, *i.e.,* it does not contain any further acetylated glycoconjugates, although it may contain additional non-acetylated glycoconjugates. In other embodiments, the sample comprises more than one acetylated glycoconjugates, e.g., both O16-EPA and O25B-EPA glycoconjugates as described herein, and, optionally contains additional non-acetylated glycoconjugates.

The inventive method therefore also allows analysis of complex samples comprising a multitude of glycoconjugates, *e.g.,* having glycans from multiple serotypes, including one or more acetylated glycoconjugates. Thus, even complex samples for release testing of a drug product, such as a multivalent glycoconjugate vaccine, may be analysed by the inventive method. It is apparent that the inventive method advances production of drug products comprising a multitude of serotypes and improves quality and safety of such complex drug products.

It is apparent that the inventive method is also suited to verify the absence of acetylated glycoconjugates, *e.g.,* blank samples.

In one embodiment, the sample comprises one carrier protein. In another embodiment, the aqueous sample comprises more than one carrier protein. The inventive method is therefore suited to analyze samples comprising multiple

different glycoconjugates, not only comprising different glycosylation patterns of the same carrier protein (different degree of glycosylation), but also differing in the carrier protein. The term carrier protein thus refers to one single carrier protein as outlined above and also to a multitude of carrier proteins, such as 2-10, *e.g.,* 2-5 different carrier proteins being present in the aqueous sample.

In certain embodiments, the sample is a production sample, *i.e.,* the sample is directly obtained from the manufacturing process of said glycoconjugate, *e.g.,* as a quality control sample, such as an in-process control sample or as a release control sample, *e.g.,* of a drug substance or of a drug product. The sample, *e.g.,* the production sample, may be provided without further treatment / preparation prior to subj ecting it to the inventive method described herein.

Step b, precipitation:

As part of the inventive method, the acetylated glycoconjugate is precipitated, whereby a pellet is obtained containing the acetylated glycoconjugate. It has been found that the acetylated glycoconjugates can be precipitated, and, thus the sample volume may be reduced. Precipitation of the acetylated glycoconjugate thus contributes to an improved overall sensitivity (*i.e.*, lower LOQ, *e.g.,* LOQ of 8 μM bound acetate, particularly bound *O*-acetate, in the sample) of the inventive method. In addition, it has been found that the acetylated glycoconjugate can be precipitated while free acetate, if present, remains in solution. Thus precipitation contributes to the removal of free acetate (if present) and other impurities (if present) from the sample.

For example, precipitation of the acetylated glycoconjugate may be performed by conducting the following steps in the order as indicated:
b-1: Adding to the sample a salt solution. Preferably the salt solution is a saturated salt solution. For example, the salt solution may be selected from saturated solutions of the following salts:

Alkali chloride salts such as NaCl, KCl, LiCl and CsCl, particularly NaCl;
Alkali earth metal chloride salts such as $MgCl_2$ and $CaCl_2$;
Alkali bromide salts such as NaBr, KBr, LiBr and CsBr, particularly NaBr; and Alkali earth metal bromide salts such as $MgBr_2$ and $CaBr_2$.

In certain embodiments, the salt solution is a saturated alkali chloride solution, particularly saturated NaCl solution (also referred to as saturated saline solution). As the skilled person understands, the salt solution is an aqueous salt solution, *i.e.,* it is a solution of the salt in water.

In certain embodiments, the salt solution is added at a volume of between 1/100 to 1/10 of the sample volume, *e.g.,* at a volume of between 1/100 to 1/50 of the sample volume, such as at a volume of 1/60 of the sample volume.

b-2: Adding to the mixture obtained after step b-1 an organic solvent, preferably selected from acetone, 2-butanone (also referred to as methyl ethyl ketone), ethanol, and isopropanol, preferably acetone, at a volume of between 3x to 10x of the sample volume.

Preferably the organic solvent, *e.g.,* acetone, is at a temperature of below -15°C, more preferably a temperature ≤ -18° C. A temperature of the organic solvent, particularly acetone, of below -15°C, *e.g.,* -15°C to -25°C, such as -18°C or -20°C, has the advantage of improved precipitation efficiency.

b-3: Optionally, the mixture obtained after step b-2 is incubated at a temperature of between 2°C to 8°C, preferably for at least 20 min. For example, the mixture obtained after b-2 may be incubated at a temperature of between 2°C to 8°C, e.g. at 2°C, 3°C, 4°C, 5°C, 6°C, 7°C or 8°C, preferably for at least 20 min, e.g. for between 20 min to 600 min, such as 20 min, 30 min, 40 min, 50 min, 60 min, 90 min, 120 min, or 180 min. Incubation of the mixture may increase precipitation efficiency; however, incubation is optional. Further, although longer incubation periods are also possible, precipitation efficiency generally does not significantly increase with longer incubation periods. Moreover, relatively short incubation periods, such as 20 min, (or no incubation) have the advantage that - in consequence - the total time required to perform the inventive method is relatively short. Thus relatively short incubation periods (or no incubation period) contribute to the aim of providing an analysis method that can provide reliable test results within one working day, i.e. within less than 8 h.

b-4: Centrifuging the mixture obtained after step b-3 to thereby obtain the pellet containing the acetylated glycoconjugate. For example, centrifugation may be performed at 9800x g for 10 min at 4°C. Although longer centrifugation periods and/or higher rates of centrifugation are possible, it has been found that good precipitation efficiency is achieved after centrifugation at 9800x g for 10 min at 4°C, without compromising integrity of the acetylated glycoconjugate.

b-5: Optionally, the pellet may be washed by repeating steps b-2 and b-3. For example the pellet may be washed 1 to 5 times, *e.g.,* 1 to 3 times, such as 2 times.
It has been found that washing the pellet is typically not required. However, in embodiments, washing the pellet improves the removal of impurities from the sample, such as free acetate or process related impurities, such as free PS.

b-6: Optionally the pellet is air-dried after centrifugation or, optionally, after washing. For example, air-drying may

be performed for 1 h at ambient temperature (about 20°C). It has been found that air-drying is typically not required. However, in embodiments, the pellet is air-dried, *e.g.,* for a period of between 5 min to 30 min, *e.g.,* 10 min in order to remove acetone that may be present of the surface of the pellet.

For example, 10 μL of saturated NaCl solution may be added to 600 μL of a test sample containing the acetylated glycoconjugate and briefly vortexed (b-1). Subsequently, about 2400 μL of pre-cooled acetone (T< -15° C) can be added, briefly vortexed (b-2), and incubated for 1h at 5°C, *e.g.,* in a fridge (b-3). After incubation, the sample may be centrifuged, e.g. at 9800x g for 10 min at 4°C to thereby obtain the pellet containing the acetylated glycoconjugate (b-4). The supernatant can be removed, *e.g.,* by decanting the supernatant.

Optionally, the pellet may be washed by adding another 2400 μL of pre-cooled acetone (T< -15° C, *e.g.,* T ≤ -18°C or T ≤ -20°C) to the pellet and centrifuging again, *e.g.,* at 9800x g for 10 minutes at ambient temperature (about 20°C). The supernatant can be removed, *e.g.,* by decanting the supernatant (b-4).

Optionally, the pellet is air-dried, *e.g.,* air-dried 1 h at ambient temperature (about 20°C).

Step c, hydrolysing *O*-acetate linkages.

As part of the inventive method, *O*-acetate linkages (i.e. acetate covalently bound to oxygen of the one or more PS) of the acetylated glycoconjugate contained in the pellet (obtained after step b, precipitation) are hydrolysed, whereby covalently bound *O*-acetate is released. As the skilled person understands, the bound *O*-acetate is cleaved from the acetylated glycoconjugate and released into the reaction mixture.

In certain embodiments, conditions are adjusted to only hydrolyse *O*-acetate linkages without hydrolysing *N*-acetate linkages, thereby releasing only acetate bound to oxygen, *e.g.,* by hydrolysis under mild alkaline conditions. Based on the present disclosure, the skilled person is able to select conditions under which only *O*-acetate linkages are hydrolysed without hydrolysing *N*-acetate linkages. For example, step c may further comprise the following steps (hydrolysis of *O*-acetate linkages without hydrolysing *N*-acetate linkages):
c-1: Dispersing the pellet containing the acetylated glycoconjugate in an alkaline solution, *e.g.,* at a pH ≥ 12, preferably an alkaline solution of NaOH or KOH. In certain embodiments, the pellet is dispersed in a solution of NaOH having a concentration of between 0.01 M to 1 M, *e.g.,* 0.05 M to 0.5 M, such as 0.1 M.

In other embodiments, the pellet is dispersed in a solution of KOH having a concentration of between 0.01 M to 1 M, *e.g.,* 0.05 M to 0.5 M, such as 0.1 M.

In certain embodiments, the pellet is dispersed in a volume of between 20 μl to 200 μl NaOH or KOH, e.g., 50 μl to 150 μl NaOH or KOH, such as 90 μl NaOH or KOH.

c-2: Incubating the mixture obtained after step c-1 (alkaline hydrolysis mixture) for a period of between 1 h to 5 h, *e.g.,* 1 h to 3 h, such as 2 h at a temperature of between 30°C to 50°C, *e.g.,* 35°C to 45°C such as 37°C. During incubation in the solution of NaOH or KOH, *O*-acetate linkages are cleaved (hydrolysed).

c-3: Quenching the incubation of step c-2 (neutralisation of the alkaline hydrolysis mixture) to thereby obtain a quenched (neutralised) hydrolysis mixture. For example, the hydrolysis mixture of step c-2 may be quenched by adding HCl or HBr, particularly HCl, *e.g.,* by adding an amount of HCl (or HBr) that is equimolar to the amount of NaOH or KOH added in step c-1.

c-4: Filtration of the quenched hydrolysis mixture obtained after step c-3 to thereby obtain a filtered test sample. During filtration, the released acetate (cleaved *O*-acetate) is separated from large molecules present in the quenched hydrolysis mixture, in particular glycoconjugate(s) and, if present, impurities such as free carrier protein or host cell protein. Accordingly, released acetate is thereby separated from previously *O*-acetylated glycoconjugates (now *O*-deacetylated glycoconjugates). For example, filtration may be performed using a molecular weight cut off (MWCO) filter, e.g. a centrifugal filter, *e.g.,* having a MWCO of 3 kDa. For example, filtration may be performed using a centrifugal MWCO filter as described above and centrifuging for 15 min to 60 min, *e.g.,* 20 min, at 14000x g at ambient temperature (about 20°C). Due to the MWCO of the filter, small molecules, particularly released acetate, pass the filter and are thus present in the filtrate, whereas large molecules, such as glycoconjugates, are retained. The filtrate containing the released acetate is then used for further analysis (quantification of released acetate).

Under the conditions described above in steps (c-1) and (c-2) (0.01 M to 1 M NaOH or KOH, incubation for 1 h to 5 h at a temperature of between 30°C to 50°C), only O-acetate linkages are cleaved (hydrolysed), while *N*-acetate linkages remain (substantially) intact.

For example, the pellet containing the acetylated glycoconjugate may be dispersed in 90 μL of 0.1 M NaOH (c-1). The thus obtained hydrolysis mixture may be incubated for 2 h at 37°C (c-2), *e.g.,* in an incubator cabinet. After incubation, the hydrolysis mixture may be quenched (neutralised) by adding 90 μL of 0.1 M HCl (c-3). Released acetate may be

separated from large molecules, particularly (now *O*-deacetylated) glycoconjugates present in the quenched hydrolysis mixture by filtration through a centrifugal MWCO filter, *e.g.,* having a MWCO of 3 kDa (*e.g.*, centrifugation for 20 min at 1400x g at ambient temperature (about 20°C); c-4). The filtrate may then be used for quantification of released *O*-acetate.

As is apparent based on the present disclosure, conditions in step c may be adjusted to hydrolyse both *O*-acetate and *N*-acetate linkages, thereby releasing acetate bound to the glycoconjugate(s) via oxygen and via nitrogen. Accordingly, in certain embodiments, in step c conditions are adjusted to hydrolyse both *O*-acetate linkages and *N*-acetate linkages, *e.g.,* by hydrolysis under harsh alkaline (*e.g.,* hydrolysis with boiling NaOH) or harsh acidic conditions, thereby releasing acetate bound to oxygen and acetate bound to nitrogen. For example:

c-1': Dispersing the pellet containing the acetylated glycoconjugate in a solution of 2 M NaOH or 2 M KOH, *e.g.,* in a volume of between 20 µl to 200 µl NaOH or KOH, *e.g.,* 50 µl to 150 µl NaOH or KOH, such as 90 µl NaOH or KOH (harsh alkaline conditions).

Alternatively, the pellet containing the acetylated glycoconjugate may be dispersed under harsh acidic conditions, e.g., in a solution having pH<1, such as in concentrated HCl or TFA, *e.g.,* in a volume of between 20 µl to 200 µl concentrated HCl or TFA, *e.g.,* 50 µl to 150 µl HCl or TFA, such as 90 µl HCl or TFA.

c-2': Incubating the mixture obtained after step c-1' (harsh alkaline hydrolysis mixture; alternatively harsh acidic hydrolysis mixtures) for a period of 6 h at 110°C. During incubation, both *O*-acetate linkages and *N*-acetate are cleaved (hydrolysed).

Steps c3' (quenching) and c4' (filtration) apply analogously as described for hydrolysis of *O*-acetate linkage without cleaving *N*-acetate linkages (steps c-3 and c-4 described above). The skilled person understands that, if hydrolysis is performed under harsh acidic conditions, the hydrolysis mixture of step c-2' may be quenched by adding an alkaline solution, *e.g.,* NaOH or KOH, *e.g.,* by adding an amount of NaOH or KOH that is equimolar to the amount of HCl or TFA added in step c-1'. If hydrolysis is performed under harsh alkaline conditions, the hydrolysis mixture of step c-2' may be quenched by adding an acidic solution, *e.g.,* HCl or HBr, *e.g.,* by adding an amount of HCl or HBr that is equimolar to the amount of NaOH or KOH added in step c-1'

<u>Step d, absolute quantification of released acetate:</u>

As part of the inventive method, absolute quantification of released acetate may be performed using a **colorimetric assay** or a **fluorometric assay** based on a calibration curve. Absolute quantification of released acetate thus includes establishing a calibration curve, *e.g.,* using free acetate as a calibration standard.

In certain embodiments, absolute quantification of released acetate is performed using a colorimetric assay. In certain embodiments this is an enzymatic colorimetric assay.

In alternative embodiments, absolute quantification of released acetate is performed using a fluorometric assay.

Surprisingly, it was found that colorimetric quantification of released acetate was more sensitive (lower LOQ) than fluorometric quantification. Nevertheless, employing fluorometric quantification according to the instant invention is still more sensitive (lower LOQ) than current methods for determining DOA of glycoconjugates, e.g. current HPAEC-CD methods. Further the inventive method still has the advantage of having a higher throughput and shorter analysis times (within 8 h) than current methods for determining DOA, while still being operable under GLP conditions satisfying the SCC criteria listed in table 1 in an economically feasible manner.

It is considered beneficial that kits for performing colorimetric or fluorometric quantification of acetate are commercially available (*e.g.*, colorimetric Acetate Assay Kit either from abcam, catalogue No.: ab204719 or from Abnova, catalogue No.: KA3764). In general, released acetate is reacted with an acetate substrate (e.g. Coenzyme A), in the presence of an acetate enzyme mix and adenosine triphosphate (ATP) to obtain an acetate intermediate (*e.g.*, acetyl-Coenzyme A). The acetate intermediate than typically reduces a colorimetric probe to a colored product, e.g. a colored product having strong absorbance at 450 nm. For example, suitable methods for quantification of acetate are described, *e.g.,* on pp. 26-28 (section 2.3.1.1) of C. Korb, Entwicklung enzymatischer Screeningverfahren (PhD thesis, 2003) and in US4035239.

Generally, absolute quantification of the released acetate (step d) comprises preparation of a reference material, *e.g.,* free acetate such as sodium acetate, and establishing a calibration curve based on said reference material. To prepare a calibration curve, aliquots of a reference material, *e.g.,* free acetate such as sodium acetate, are measured using the same assay, particularly the enzymatic colorimetric assay, that is used for analysing the test sample. For example, a calibration curve may be established based on aliquots with a free acetate concentration, *e.g.,* a sodium acetate concentration, of 0 µM, 20 µM, 40 µM, 60 µM, 80 µM and 100 µM, thereby establishing a calibration curve between 0 µM and 100 µM acetate. As the skilled person understands, the calibration curve can be used for absolute quantification of released acetate in the filtered test sample obtained after step c.

In certain embodiments, step d thus further comprises the following steps:

d-1: Preparing aliquots of a solution containing a defined concentration of the reference material, *e.g.,* free acetate, *e.g.,* aliquot having a different concentration of reference material.

d-2: Contacting each aliquot of the reference material, *e.g.,* free acetate, with a mixture comprising:

- an acetate enzyme mix
- an acetate substrate Mix
- ATP
- a colorimetric probe.

d-3: Measuring the absorbance of each aliquot at a wavelength adapted to the colorimetric probe to thereby establish the calibration curve.

As the skilled person understands, "a wavelength adapted to the colored probe" refers to a wavelength at which a colored product obtained from reaction of the colored probe can be distinguished from the colored probe.
In certain embodiments, step d further comprises absolute quantification of the released acetate after step c (hydrolysis of *O*-acetate linkages) based on the calibration curve according to the following steps:

d-4: Contacting the filtered test sample obtained after step c with a mixture comprising:

- an acetate enzyme mix
- an acetate substrate Mix
- ATP
- a colorimetric probe.

d-5: Measuring absorbance at a wavelength adapted to the colorimetric probe.
d-6: Comparing the measured absorbance of the test sample with the calibration curve.

In certain embodiments, in order to account for background signal, a reference sample is measured in addition to the (test) sample. As used herein, the reference sample is a sample not comprising an acetylated glycoconjugate but otherwise being identical to the (test) sample. Further, it is understood that the reference sample is subjected to the same treatment as the (test) sample. The signal measured for the reference sample (absolute quantification of released acetate) is then subtracted from the signal measured for the (test) sample. Although, additionally measuring a reference sample may (slightly) improve quantification, measuring a reference sample is typically not required in order to obtain reliable absolute quantification of bound acetate, particularly bound *O*-acetate.

<u>Step e, determining DOA</u>

As the skilled person understands based on the present disclosure, the concentration of released acetate in the sample can be determined by comparison with the calibration curve (absolute quantification of released acetate). DOA of an acetylated glycoconjugate may then be determined based on the absolute quantification of the released acetate and the known concentration of acetylated glycoconjugate (measured as concentration of PS component), e.g., according to the following formula:

$$DOA = \frac{[released\ acetate] \times [mol.weight\ repeating\ unit]\ \times 100\%}{[acetylated\ glycoconjugate] \times [O - acetate\ groups\ per\ RU]}$$

For example, molecular weight of an RU of *E. coli* 016 antigen PS = 715.7 g/mol (calculated based on structure according to table 2). A known concentration of 120 μg/mL of acetylated glycoconjuagte of *E. coli* 016 antigen PS covalently bound to EPA carrier protein (measured as concentration of PS component) thus corresponds to 168 μmol/L of *E. coli* 016 antigen PS RUs. Each *E. coli* 016 RU may contain one covalently bound *O*-acetate group (cf. table 2). Accordingly, a released acetate concentration of 168 μmol/L corresponds to 100% DOA as calculated below:

$$DOA = 100\% = \frac{[168\ \mu\mathrm{mol/L}] \times [715.7\ g/mol]\ \times 100\%}{[120\ \mu g/\mathrm{mL}] \times 1}$$

If in step c conditions are adjusted to hydrolyse both *O*-acetate and *N*-acetate linkages, thereby releasing acetate bound to oxygen and acetate bound to nitrogen, both degree of *O*-acetylation and degree of *N*-acetylation may be determined. If both degree of O-acetylation and degree of *N*-acetylation should be determined, steps b, c and d described

above may be adapted as follows and an additional step f included as follows:

Step b: The sample is divided into a first aliquot to measure *O*-acetylation and a second aliquot to measure both *O*-acetylation and *N*-acetylation.
Step c: For the first aliquot, conditions are adjusted to only hydrolyse O-acetate linkages without hydrolysing *N*-acetate linkages, thereby releasing only acetate bound to oxygen, e.g. as described above. For the second aliquot conditions are adjusted to hydrolyse both *O*-acetate linkages and *N*-acetate linkages, thereby releasing acetate bound to oxygen and acetate bound to nitrogen, *e.g.,* as described above (*e.g.,* cf. steps c1' - c4').
Step d: Absolute quantification of the released acetate is performed for the first aliquot to determine *O*-acetylation and for the second aliquot to determine both *O*-acetylation and *N*-acetylation (same protocol for *O*-acetylation and *N*-acetylation; *e.g.,* as described above).
Additional step f: Calculating the degree of *O*-acetylation and *N*-acetylation by subtracting the concentration of released acetate measured for the first aliquot from the concentration of released acetate measured for the second aliquot.

Examples

To further illustrate the invention, the following examples are provided. These examples are provided with no intend to limit the scope of the invention.

Example 1; DOA of *E. coli* O16-EPA glycoconjugate present in a mixture of glycoconjugates

Test sample:

In this example, the (test) sample contained the following components:

- *E. coli* O16-EPA glycoconjugate (bioconjugate; EPA carrier protein having SEQ ID NO: 1 of WO 2022/214620 glycosylated with *E. coli* 016 antigen PS; *O*-acetylated, cf. table 2) at a concentration of 7.7 μg/ml (based on the concentration of *E. coli* 016 antigen PS);
- *E. coli* O1-EPA glycoconjugate (not *O*-acetylated), at a concentration of 22.4 μg/ml (based on the concentration of A. *coli* O1 antigen PS)
- Sample matrix: 6.19 mM $KH_2PO_4$, 3.81 mM $Na_2HPO_4$, 5% (w/w) sorbitol, 10 mM methionine, and 0.02% (w/w) polysorbate-80 at pH 7.0

The concentration of bound *O*-acetate was previously known based on quantification of bound acetate via HPAEC-CD. The test sample was then diluted to a concentration corresponding to 8.4 μM bound *O*-acetate (also referred to as "nominal value of bound O-acetate"; corresponds to 7.7 μg/ml of *E. coli* 016 antigen PS bound to EPA carrier protein with DOA = 78%).

In addition, in order to account for background signal, a reference sample (for background subtraction), *i.e.,* a sample not comprising *E. coli* O16-EPA glycoconjugate but otherwise being identical to the test sample (*i.a.* comprising *E. coli* O1-EPA glycoconjugate at a concentration of 22.4 μg/ml PS).

Chemicals:

- Ultrapure water (MilliQ)
- 1 M HCl (Honeywell, 35328-1L); used to prepare 0.1 M HCl
- 1 M NaOH (Honeywell, 35256-1L); used to prepare 0.1 M NaOH
- 0.5 M NaCl (VWR, 85812.290)
- Sodium chloride ≥ 99.5 % Microbiology grade (VWR, 33614.265)
- Acetone in glass bottle Spectronorm ≥ 99.8 % (VWR, 84700.320) precooled in freezer (stored at ≤ -18° C)
- Sodium acetate solution 1 M USP TS 34 (VWR, 85371.180); used to prepare 1 mM sodium acetate solution
- Saturated sodium chloride (NaCl) solution: 20 g ± 1g of sodium chloride was added to a 50 mL Falcon tube and filled up to 50 mL with water MilliQ at room temperature (RT). Suspension was mixed repeatedly for 15 seconds on a vortex mixer every 10 minutes during one hour at RT. Only the clear supernatant was used for experiments, while the undissolved NaCl remained on the bottom of the Falcon tube.
- Abnova Acetate Assay Kit (catalogue No.: KA3764)

Calibration standards:

Sodium acetate calibration standards were prepared at the following concentrations from the 1 mM sodium acetate solution: 0 μM, 20 μM, 40 μM, 60 μM, 80 μM, and 100 μM

Procedure - sample preparation and acetate quantification:

The test sample and the reference sample were prepared for analysis as follows: 600 μL of each of the test sample and the reference sample were used. In the following, the sample preparation is described for a single sample (sample preparation being identical for the test sample and the reference sample).

Precipitation: The sample was transferred into a 15 mL Falcon tube and 10 μL of saturated NaCl solution was added. The sample was briefly vortexed and incubated in the fridge (2 - 8°C) for 1 h. Then the incubated sample was taken from the fridge and immediately centrifuged at 9800x g for 10 minutes at 4°C. A pellet was visible at the bottom of the tube. The supernatant was decanted by pipetting the liquid out of the tube until about 2 mm above the visible pellet, without slurrying-up the pellet.

Then the sample was washed once by adding another 2400 μL of pre-cooled Acetone (T≤-18° C) to the tube. The sample was then centrifuged again at 9800x g for 10 minutes at RT. A pellet was visible at the bottom of the tube. The supernatant was decanted by pipetting the liquid out of the tube until about 2 mm above the visible pellet. The sample (pellet) was then air-dried for 1 h at RT.

Hydrolysis: 90 μL of 0.1 M NaOH was added to the pellet and the Falcon tube was closed firmly with a screwcap. The sample was vortexed vigorously for about 10 seconds and incubated for 2 h at 37°C in an incubator cabinet. During incubation, the sample was vortexed for about 5 seconds every 30 minutes to ensure that the pellet dissolves during incubation. After 2 h incubation at 37°C, the sample was taken from the incubator cabinet and centrifuged briefly to recover all condensated liquid droplets. The hydrolysis mixture was then quenched (neutralised) by adding 90 μL of 0.1 M HCl and the sample was briefly mixed on a vortex mixer. Then the sample was spun down (1 min at 1000x g at RT). The sample was then filtered using a centrifugal filter (MWCO = 3kDa; centrifuged for 20 min at 14000x g at RT). The centrifugal filters were discarded and the filtrate was briefly vortexed (filtered sample).

Quantification (96 well plate; enzymatic colorimetric assay):

The following master mix was prepared from reagents of the commercially available Abnova Acetate Assay Kit (catalogue no.: KA3764) in a 5 mL tube, which was cooled in an ice-bucket. The master mix was prepared according to Table 3 by multiplying the given amounts of the respective reagents with the number of wells, plus 8 wells as reserve (excess of master mix): For calibration, 12 wells were used (six calibration standards as listed above). In addition, two wells were used for each of the test sample and the reference sample (each sample / calibration standard was measured in duplicates):

Table 3. Reagent mixture for acetate quantification

| Reagent | Volume per well [μL] |
|---|---|
| Acetate assay buffer | 42 |
| Acetate enzyme mix | 2 |
| Acetate substrate mix | 2 |
| ATP | 2 |
| Colorimetric probe | 2 |

50 μL of filtered sample and 50 μL of the master mix according to table 3 were added to each well on a 96 well plate (two wells per sample for duplicate measurement; 12 calibration samples). The 96 well plate was shaken for 5 seconds within a spectrophotometric plate reader (SpectraMax iD5 Spectrophotometer from Molecular Devices) and absorbance was measured for 40 minutes at 450 nm.

The concentration of released acetate in the sample was determined by comparison of the absorbance value of the sample with the calibration curve (calibration curve shown in Fig. 2), taking into account the absorbance signal measured for the reference sample (absolute quantification of released acetate).

In addition, DOA of the O16-EPA glycoconjugate was determined based on the absolute quantification of the released acetate and the known concentration of acetylated glycoconjugate according to the following formula:

$$DOA = \frac{[released\ acetate] \times [mol.weight\ repeating\ unit]\ \times 100\%}{[acetylated\ glycoconjugate] \times [O - acetate\ groups\ per\ RU]}$$

Further, the recovery rate was calculated according to the following formula:

$$Recovery = \frac{[released\ acetate]\ \times 100\%}{[nominal\ value\ of\ bound\ O - acetate]}$$

In this example, the concentration of released acetate was found to be 8.88 μM (average of duplicate measurements). Accordingly, DOA of the O16-EPA glycoconjugate was found to be 82.5% (average of duplicate measurements). Results are shown in table 4. The calibration curve is shown in Fig. 1.

Table 4. Results of bound O-acetate quantification; O16-EPA glycoconjugate (nominal bound *O*-acetate concentration of 8.4 μM)

| Sample / calibration standard | Absorbance at 450 nm | Released acetate [μM] | Recovery of bound acetate | DOA of 016-EPA |
|---|---|---|---|---|
| Calibration 1 (0 μM acetate), duplicate 1 | 0.247 | n.a. | n.a. | n.a. |
| Calibration 1 (0 μM acetate), duplicate 2 | 0.247 | n.a. | n.a. | n.a. |
| Calibration 2 (20 μM acetate), duplicate 1 | 0.375 | n.a. | n.a. | n.a. |
| Calibration 2 (20 μM acetate), duplicate 2 | 0.361 | n.a. | n.a. | n.a. |
| Calibration 3 (40 μM acetate), duplicate 1 | 0.507 | n.a. | n.a. | n.a. |
| Calibration 3 (40 μM acetate), duplicate 2 | 0.499 | n.a. | n.a. | n.a. |
| Calibration 4 (60 μM acetate), duplicate 1 | 0.636 | n.a. | n.a. | n.a. |
| Calibration 4 (60 μM acetate), duplicate 2 | 0.634 | n.a. | n.a. | n.a. |
| Calibration 5 (80 μM acetate), duplicate 1 | 0.767 | n.a. | n.a. | n.a. |
| Calibration 5 (80 μM acetate), duplicate 2 | 0.765 | n.a. | n.a. | n.a. |
| Calibration 6 (100 μM acetate), duplicate 1 | 0.882 | n.a. | n.a. | n.a. |
| Calibration 6 (100 μM acetate), duplicate 2 | 0.888 | n.a. | n.a. | n.a. |
| Sample (O16-EPA), duplicate 1 (without background subtraction) | 0.362 | 10.90 | n.a. | n.a. |
| Sample (O16-EPA), duplicate 2 (without background subtraction) | 0.363 | 10.99 | n.a. | n.a. |
| Reference sample (O1-EPA in Matrix without O16-EPA) | 0.287 | n.a. | n.a. | n.a. |
| Reference sample (O1-EPA in Matrix without O16-EPA) | 0.273 | n.a. | n.a. | n.a. |
| Sample (O16-EPA), duplicate 1, after background subtraction | n.a. | 8.83 | 105% | 82.1% |

(continued)

| Sample / calibration standard | Absorbance at 450 nm | Released acetate [μM] | Recovery of bound acetate | DOA of 016-EPA |
|---|---|---|---|---|
| Sample (O16-EPA), duplicate 2, after background subtraction | n.a | 8.92 | 106% | 82.9% |
| n.a. = not applicable | | | | |

This example (inventive method) shows that a sample containing 8.4 μM bound *O*-acetate in the starting material, *i.e.,* the concentration of bound *O*-acetate in the test sample prior to sample preparation, is quantifiable via the inventive method (precision = 4%, RSD; recovery = 102%).

42 samples (e.g. different production batches for release control) can be measured in parallel in duplicates (84 wells) along with the calibration curve (6 calibration standards measured in duplicates). The whole method was completed within less than 8 h.

The above experiment was repeated with O16-EPA test samples containing higher concentrations of bound *O*-acetate (but otherwise being identical to the test sample described above), and the recovery rates (bound acetate) were determined. Additional test samples having the following nominal concentrations of bound *O*-acetate (bound *O*-acetate measured by HPAEC-CD) were additionally tested: 16.9 μM, 21.1 μM, 33.8 μM, 42.2 μM and 52.8 μM (corresponding to a DOA of 40%, 60%, 80%, 100% and 120%, respectively).
Results of these additional test samples are shown in table 5.

Table 5. Results of additional bound acetate / DOA measurements; O16-EPA glycoconjugate (nominal bound *O*-acetate concentration > 8.4 μM)

| Sample / calibration standard | Absorbance at 450 nm | Released acetate (background subtracted) [μM] | Recovery of Bound Acetate | DOA of 016-EPA |
|---|---|---|---|---|
| Sample (O16-EPA) 16.9 μM bound acetate, duplicate 1 | 0.452 | 17.75 | 105% | 42.0% |
| Sample (O16-EPA) 16.9 μM bound acetate, duplicate 2 | 0.467 | 19.08 | 113% | 45.2% |
| Sample (O16-EPA) 21.1 μM bound acetate, duplicate 1 | 0.498 | 22.26 | 105% | 63.3% |
| Sample (O16-EPA) 21.1 μM bound acetate, duplicate 2 | 0.510 | 23.39 | 111% | 66.5% |
| Sample (O16-EPA) 33.8 μM bound acetate, duplicate 1 | 0.647 | 36.91 | 109% | 87.4% |
| Sample (O16-EPA) 33.8 μM bound acetate, duplicate 2 | 0.648 | 37.01 | 109% | 87.6% |
| Sample (O16-EPA) 42.2 μM bound bcetate, duplicate 1 | 0.751 | 47.07 | 112% | 111.5% |
| Sample (O16-EPA) 42.2 μM bound acetate, duplicate 2 | 0.736 | 45.69 | 108% | 108.3% |
| Sample (O16-EPA) 52.8 μM bound acetate, duplicate 1 | 0.817 | 53.24 | 101% | 121.0% |
| Sample (O16-EPA) 52.8 μM bound acetate, duplicate 2 | 0.863 | 57.54 | 109% | 130.8% |

In addition, a 9-valent glycoconjugate composition, produced during a development campaign, was analysed as described above. The 9-valent composition contained glycoconjugates of *E. coli* O-antigen polysaccharides O1A, O2, O4, O6A, 015, 016, O18A, O25B and O75, each *E. coli* O-antigen polysaccharide being individually coupled to EPA carrier protein. In this composition, 016 and O25B antigen polysaccharides are O-acetylated. A released acetate concentration of 45.1 μM was found (average of duplicate measurements; corresponds to an average DOA of 80.5%). The presence of

additional glycoconjugates did not disturb quantification, which shows that the method of the invention is also suitable for complex mixtures of glycoconjugates.

Example 2; comparative example HPAEC-CD (current gold standard); DOA of *E. coli* O6-EPA glycoconjugate

In this comparative example, sensitivity of HPAEC-CD, *i.e.,* the current gold standard for acetate quantification, was evaluated. A calibration curve was established with free sodium acetate in $H_2O$ and the signal-to-noise ratio (S/N) was determined based on spiked samples containing a pharmaceutically acceptable buffer (matrix) spiked with sodium acetate during sample preparation. The spiked samples served as a surrogate for an *O*-acetylated EPA glycoconjugate.

Spiked samples for HPAEC-CD measurements contained the following components:

- Matrix: 6.19 mM $KH_2PO_4$, 3.81 mM $Na_2HPO_4$, 5% (w/w) sorbitol, 10 mM methionine, and 0.02% (w/w) polysorbate-80 at pH 7.0;
- after desalting step, different concentrations of sodium acetate were added (spiked in).

Preparation of spiked samples:

Each sample (prior to adding different concentrations of spiked sodium acetate) was desalted by passing a sample volume of 500 µL through a Zeba resin (size exclusion chromatography resin). From each desalted sample, 160 µL sample volume was transferred into two separate Eppendorf tubes (duplicates) and 20 µL of 0.1 M NaOH, 20 µL of propionate (internal standard), and varying amounts of sodium acetate solution were added (2.6 µL of either 100 µg/ml or 200 µg/ml "sodium acetate spike-solution" were added to obtain a spiked sample containing either 42.4 µM acetate or 84.7 µM acetate as surrogate for bound *O*-acetate. The spiked samples were incubated for 4 h at 37°C (mimics hydrolysis of *O*-acetate linkages). After incubation, the spiked samples were transferred onto a 3 kDa MWCO filter and centrifuged for 20 min at 14000x g at 4°C. For each spiked sample, 180 µL of filtrate (containing the spiked acetate) was transferred into a 0.3 mL plastic vial and closed with a cut cap (Y-slit septum).

In addition, sodium acetate calibration standards in $H_2O$ were prepared at the following concentrations from a 1000 mg/L sodium acetate solution: 2.5 µg/mL (42.4 µM), 5 µg/mL(84.7 µM), 7.5 µg/mL (127.1 µM), 10 µg/mL (169.5 µM), 12.5 µg/mL (254.2 µM).

HPAEC-CD was used to quantify free acetate (surrogate for released acetate) in the spiked samples and the sodium acetate calibration standards (injection volume: 25µl). HPAEC-CD was performed on an anion exchange column in analogy to the method described by Kao et al. [Vaccine, 2004 Jan 2;22(3-4):335-44].

Calculation of acetate content in spiked samples:

The spiked acetate content was quantified by comparing the peak area of the acetate peak of the spiked sample with the peak area of the sodium acetate calibration standards. Normalisation to the peak area of the internal standard (propionate) was performed in order to account for injection-to-injection variability. Results are shown in table 6.

Table 6. Results of acetate quantification via HPAEC-CD

| Sample / calibration standard | Peak area [µS*min] | Recovered acetate [µg/mL] | S/N Ratio of Acetate peak |
|---|---|---|---|
| Calibration 1 (2.5 µg/mL (42.4 µM), duplicate 1 | 0.1577 | n.a. | n.a. |
| Calibration 1 (2.5 µg/mL (42.4 µM), duplicate 2 | 0.1502 | n.a. | n.a. |
| Calibration 2 (5 µg/mL (84.7 µM), duplicate 1 | 0.2970 | n.a. | n.a. |
| Calibration 2 (5 µg/mL (84.7 µM), duplicate 2 | 0.2973 | n.a. | n.a. |
| Calibration 3 (7.5 µg/mL (127.1 µM), duplicate 1 | 0.4435 | n.a. | n.a. |
| Calibration 3 (7.5 µg/mL (127.1 µM), duplicate 2 | 0.4400 | n.a. | n.a. |
| Calibration 4 (10 µg/mL (169.5 µM), duplicate 1 | 0.5835 | n.a. | n.a. |
| Calibration 4 (10 µg/mL (169.5 µM), duplicate 2 | 0.5696 | n.a. | n.a. |
| Calibration 5 (12.5 µg/mL (254.2 µM), duplicate 1 | 0.7039 | n.a. | n.a. |
| Calibration 5 (12.5 µg/mL (254.2 µM), duplicate 2 | 0.6570 | n.a. | n.a. |
| Slope of linear regression curve | 0.0549 | n.a. | n.a. |

(continued)

| Sample / calibration standard | Peak area [µS*min] | Recovered acetate [µg/mL] | S/N Ratio of Acetate peak |
|---|---|---|---|
| Standard Deviation of intercept of linear regression curve | 0.0087 | n.a. | n.a. |
| Spiked sample 1 (42.4 µM acetate) | 0.1518 | 2.49 | 10.6 |
| Spiked sample 2 (42.4 µM acetate) | 0.1456 | 2.38 | 10.6 |
| Spiked sample 3 (42.4 µM acetate | 0.1565 | 2.57 | 11.0 |
| Spiked sample 4 (42.4 µM acetate) | 0.1591 | 2.62 | 11.0 |
| Spiked sample 5 (84.7 µM) | 0.2932 | 5.06 | 19.5 |
| Spiked sample 6 (84.7 µM acetate) | 0.2864 | 4.94 | 19.5 |
| n.a. = not applicable; Four samples spiked with 42.4 µM sodium acetate (S/N close to 10) and two samples spiked with 84.7 µM sodium acetate were analyzed. | | | |

LOQ (concentration of bound *O*-acetate in the starting material, *i.e.,* prior to sample preparation) for determining bound *O*-acetate in this comparative example using the HPAEC-CD based method was found to be 42.4 µM bound *O*-acetate, based on an S/N threshold of 10.

Measurement of six samples (when measured in duplicates) along with a calibration curve (5 calibration standards measured in duplicates) takes 18 h.

Description of the drawings

Figure 1: Workflow scheme.

I: Sample comprising acetylated glycoconjugate; II: Precipitation of acetylated glycoconjugate, *e.g.,* by addition of saturated saline and acetone; III: optional incubation; IV: centrifugation; V: optional washing step, *e.g.* removing supernatant and washing with acetone; VI: optional removal of washing solution, *e.g.,* acetone; VII: hydrolysis of O-acetate linkages; VIII: optional filtration; IX: absolute quantification of released acetate using colorimetric assay or fluorometric assay

Figure 2: Calibration curve. Sodium acetate calibration standards were prepared at the following concentrations: 0 µM, 20 µM, 40 µM, 60 µM, 80 µM, and 100 µM. Calibration was performed as described in example 1.

## Claims

**1.** A method for absolute quantification of covalently bound acetate of an acetylated glycoconjugate in a sample,

wherein the acetylated glycoconjugate comprises one carrier protein and one or more polysaccharides covalently bound to said carrier protein, wherein one or more acetate groups are covalently bound to oxygen of the one or more polysaccharides,
the method comprising the following steps:

a) providing a sample containing the acetylated glycoconjugate; then
b) precipitating the acetylated glycoconjugate, to thereby obtain a pellet containing the acetylated glycoconjugate; then
c) hydrolysing *O*-acetate linkages of the acetylated glycoconjugate contained in the pellet, to thereby release covalently bound acetate; then
d) absolute quantification of the released acetate using a colorimetric assay or a fluorometric assay based on a calibration curve.

**2.** The method according to claim 1, wherein the carrier protein is selected from the group consisting of detoxified Exoprotein A of *Pseudomonas aeruginosa* (EPA), *Escherichia coli* flagellin (FliC), CRM197, maltose binding protein (MBP), Diphtheria toxoid, Tetanus toxoid, detoxified hemolysin A of *Staphylococcus aureus,* clumping factor A,

clumping factor B, *Escherichia coli* heat labile enterotoxin, detoxified variants of *Escherichia coli* heat labile enterotoxin, Cholera toxin B subunit (CTB), cholera toxin, detoxified variants of cholera toxin, *Escherichia coli* Sat protein, the passenger domain of *Escherichia coli* Sat protein, *Streptococcus pneumoniae* Pneumolysin, Keyhole limpet hemocyanin (KLH), *Pseudomonas aeruginosa* PcrV, outer membrane protein of *Neisseria meningitides* (OMPC), and protein D from non-typeable *Haemophilus influenza.*

**3.** The method according to claim 2, wherein the carrier protein is a detoxified Exoprotein A of *Pseudomonas aeruginosa* (EPA).

**4.** The method according to any one of claims 1 to 3, wherein the one or more polysaccharides are O-antigen polysaccharides specific to a Gram-negative bacterium, preferably selected from the group consisting of *Escherichia* and *Shigella,* more preferably *E. coli.*

**5.** The method according to any one of claims 1 to 4, wherein the one or more polysaccharides each comprise 1-100, preferably 5 - 20, repeating units.

**6.** The method according to any one of claims 1 to 5, wherein the polysaccharide is an *E. coli* O-antigen polysaccharide selected from *E. coli* serotypes 016 and O25B.

**7.** The method according to any one of claims 1 to 6, wherein the sample further comprises:

- an aqueous matrix, said matrix optionally including one or more of buffers, inorganic salts, free acetate, sugar alcohols, and non-ionic surfactants;
- optionally carrier protein free of polysaccharides;
- optionally polysaccharides not bound to carrier protein ("free PS");
- optionally non-related proteins.

**8.** The method according to claim 7, wherein the sample comprises a multitude of different glycoconjugates, preferably 2 - 20 different glycoconjugates, said different glycoconjugates differing in the polysaccharide components and/or in the carrier proteins.

**9.** The method according to any one of claims 1 to 8, wherein the sample is a production sample.

**10.** The method according to any one of claims 1 to 9, wherein step b further comprises the following steps:

b-1) adding to the sample a salt solution, preferably a saturated salt solution; then
b-2) adding to the mixture obtained after b-1 an organic solvent, preferably selected from acetone, 2-butanone, ethanol, and isopropanol, at a volume of between 3x to 10x of the sample volume, preferably wherein the organic solvent is at a temperature of below -15°C; then
b-3) optionally incubating the mixture obtained after b-2 at a temperature of between 2°C to 8°C, preferably for at least 20 min; then
b-4) centrifuging the mixture obtained after b-3) to thereby obtain the pellet containing the acetylated glycoconjugate; then
b-5) optionally washing the pellet, preferably by repeating steps b-2 and b-3; then
b-6) optionally air-drying the pellet.

**11.** The method according to any one of claims 1 to 10, wherein in step c conditions are adjusted to only hydrolyse *O*-acetate linkages without hydrolysing *N*-acetate linkages, thereby releasing only acetate bound to oxygen.

**12.** The method according to any of one of claims 1 to 11, wherein step c further comprises the following steps:

c-1) dispersing the pellet containing the acetylated glycoconjugate in an alkaline solution, preferably an alkaline solution of NaOH or KOH, more preferably NaOH or KOH at a concentration of between 0.01 M to 1 M; then
c-2) incubating the mixture obtained after c-1 for a period of between 1 h to 5 h at a temperature of between 30°C to 50°C; then
c-3) quenching the incubation of step c-2 to thereby obtain a quenched hydrolysis mixture; then
c-4) filtration of the quenched hydrolysis mixture obtained after step c-3.

**13.** The method according to any one of claims 1 to 12, wherein in step d, the released acetate is quantified using the colorimetric assay.

**14.** The method according to claim 13, wherein the colorimetric assay is an enzymatic colorimetric assay.

**15.** The method according to any one of claims 1 to 14, wherein the concentration of the acetylated glycoconjugate in the sample is known, and the method further comprises step e) determining the degree of acetylation based on the absolute quantification of the released acetate and the known concentration of acetylated glycoconjugate.

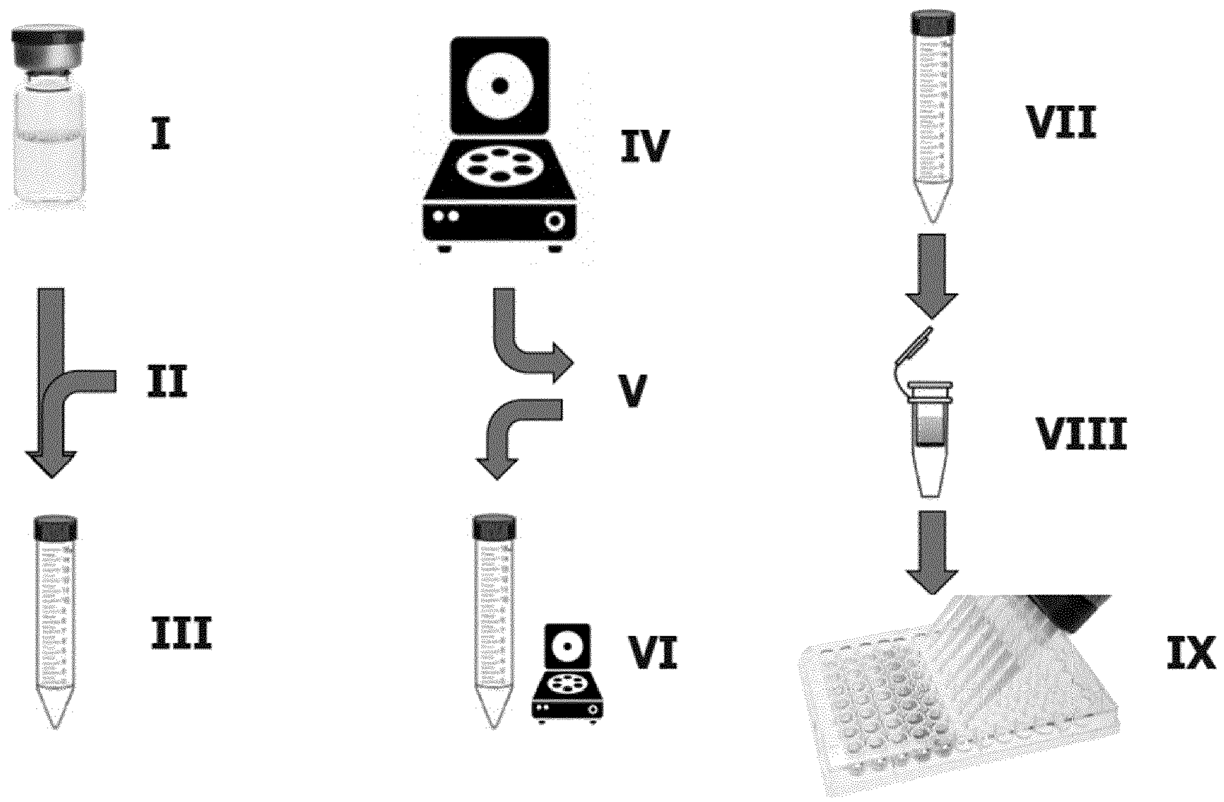


FIG. 1

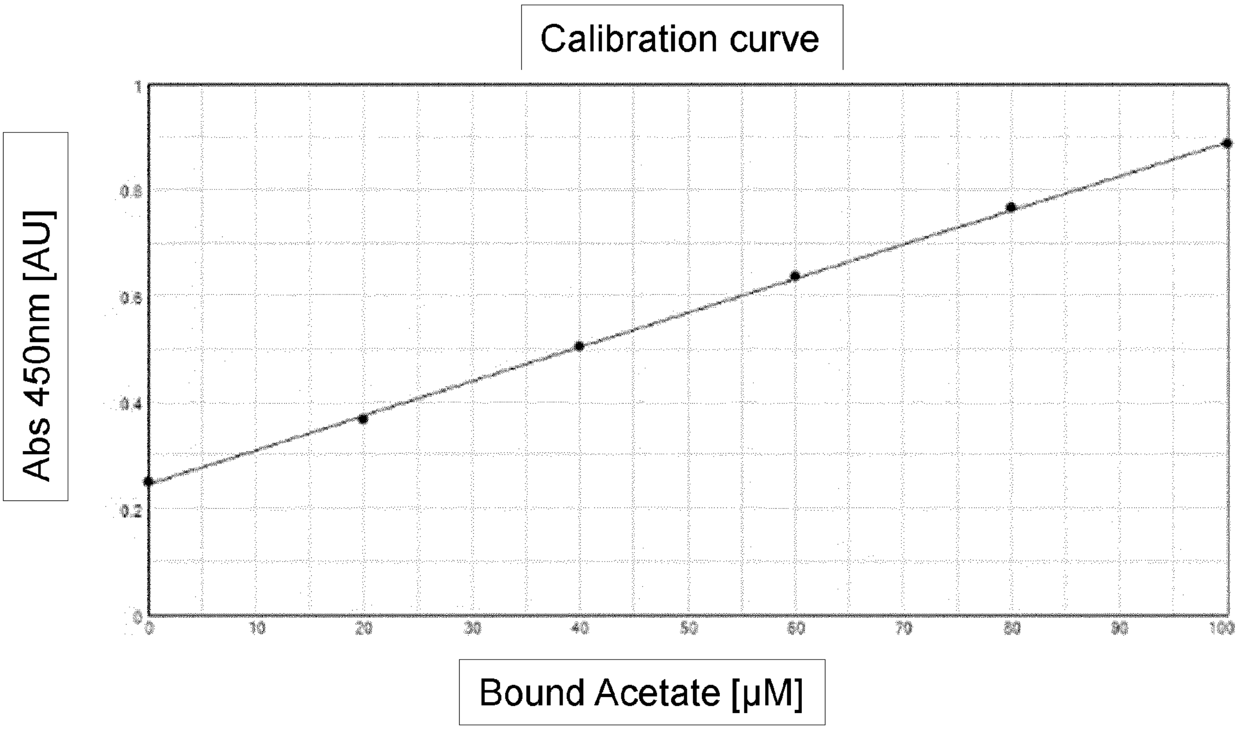
Calibration curve
Abs 450nm [AU]
1
0.8
0.6
0.4
0.2
0
0
10
20
30
40
50
60
70
80
90
100
Bound Acetate [µM]

FIG. 2

European Patent Office

## EUROPEAN SEARCH REPORT

Application Number
EP 23 17 8621

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BHULLAR KASH A ET AL: "Assessing the quantification of acetylation in konjac glucomannan via ATR-FTIR and solid-state NMR spectroscopy", CARBOHYDRATE POLYMERS, APPLIED SCIENCE PUBLISHERS , LTD BARKING, GB, vol. 291, 26 May 2022 (2022-05-26), XP087092719, ISSN: 0144-8617, DOI: 10.1016/J.CARBPOL.2022.119659 [retrieved on 2022-05-26] * the whole document * * In particular: Title; Abstract; Materials and methods. * ----- | 1-15 | INV. G01N33/68 |
| A | KAO G ET AL: "Quantification of O-acetyl, N-acetyl and phosphate groups and determination of the extent of O-acetylation in bacterial vaccine polysaccharides by high-performance anion-exchange chromatography with conductivity detection (HPAEC-CD)", VACCINE, ELSEVIER, AMSTERDAM, NL, vol. 22, no. 3-4, 2 January 2004 (2004-01-02), pages 335-344, XP004479353, ISSN: 0264-410X, DOI: 10.1016/J.VACCINE.2003.08.008 * the whole document * * In particular: Title; Abstract; Materials and methods. * ----- | 1-15 | TECHNICAL FIELDS SEARCHED (IPC)<br>G01N |

The present search report has been drawn up for all claims

1

| Place of search | Date of completion of the search | Examiner |
|---|---|---|
| The Hague | 26 October 2023 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015124769 A
- WO 2017035181 A
- WO 2020191082 A1
- WO 2022214620 A
- WO 2009104074 A
- WO 2020191082 A
- WO 2018077853 A
- WO 2022058945 A
- WO 2021219530 A
- US 4035239 A


### Non-patent literature cited in the description


- **POOLMAN** ; **WACKER**. *J. Infect. Dis.*, 2016, vol. 213 (1), 6-13
- **KAO et al.** *Vaccine*, 02 January 2004, vol. 22 (3-4), 335-44
- **HESTRIN et al.** *J Biol Chem*, 1949, vol. 180, 249-61
- **BUNDLE et al.** *J Biol Chem*, 1974, vol. 249 (7), 2275-81
- **BHATTACHARJEE et al.** *Can J Biochem*, 1976, vol. 54 (1), 1-8
- **J. S. ROHRER**. Carbohydrate Analysis by Modern Liquid Phase Separation Techniques. 2021, 157-207